# EUROPÄISCHE PATENTSCHRIFT

(11) **EP 0 671 384 B1**
(45) Veröffentlichungstag und Bekanntmachung des Hinweises auf die Patenterteilung: **03.11.1999**
(21) Anmeldenummer: 95102923.0
(22) Anmeldetag: 02.03.1995
(51) Int. Cl.: C07C 255/46, C07C 253/10

(54) **Verfahren zur Herstellung von 3-Cyano-3,5,5-trimethylcyclohexanon**
Process for the preparation of 3-cyano-3,5,5,-trimethyl-cyclohexanone
Procédé pour la préparation de 3-cyano-3,5,5-triméthyl-cyclohexanone

(30) Priorität: 07.03.1994 DE 4407487
(43) Veröffentlichungstag der Anmeldung: 13.09.1995
(73) Patentinhaber: BASF AKTIENGESELLSCHAFT, 67056 Ludwigshafen (DE)
(72) Erfinder: Mundinger, Klaus, Dr., D-67117 Limburgerhof (DE); Laqua, Gerhard, Dr., D-68167 Mannheim (DE); Witzel, Tom, Dr., D-67069 Ludwigshafen (DE); Merger, Franz, Dr., D-67227 Frankenthal (DE)

(56) Entgegenhaltungen:
- EP-A- 0 502 707
- US-A- 5 011 968
- US-A- 5 183 915
- CHEMICAL ABSTRACTS, vol. 105, no. 5, 4. August 1986, Columbus, Ohio, US; abstract no. 42383u, K. KONDO ET. AL. '3-Cyano-3,5,5-trimethylcyclohexanone.' Seite 698 ;Spalte 1 ; & JP-A-61 033 157 (NIPPON CHEMICALS CO. LTD.)

## Beschreibung

Die vorliegende Erfindung betrifft ein Verfahren zur Herstellung von 3-Cyano-3,5,5-trimethylcyclohexanon (Isophoronnitril) durch Anlagerung von Cyanwasserstoff an Isophoron in Gegenwart von katalytischen Mengen eines quartären Ammoniumsalzes bei erhöhten Temperaturen.

Aus der US-A-5 011 968 ist ein Verfahren zur Herstellung von 3-Cyano-3,5,5-trimethylcyclohexanon aus Isophoron und Cyanwasserstoff in Gegenwart von katalytischen Mengen eines quartären Ammonium- bzw. Phosphoniumhydroxidkatalysators bekannt.

Aus der EP-A-502 707 ist bekannt, daß quartäre Ammoniumhydroxide aufgrund ihrer starken Basizität zu HCN-Polymerisation und zu Oligomerisierung des Isophorons führen.

Aus der US-A-5 183 915 ist ein Verfahren zur Herstellung von 3-Cyano-3,5,5-trimethylcyclohexanon aus Isophoron und Cyanwasserstoff in Gegenwart von quartären Ammoniumcyanid- und Phosphoniumcyanid-katalysatoren bekannt. Diese Katalysatoren sind jedoch teuer und die 3-Cyano-3,5,5-trimethylcyclohexanon-Ausbeuten sind verbesserungsbedürftig.

In EP-A-502 707 wird die Verwendung von quartären Ammonium- und Phosphoniumhalogeniden unter Zusatz einer basischen Komponente beschrieben. In Abwesenheit von Wasser wird hierbei eine heterogene Reaktionsmischung erhalten, die einen kontinuierlichen Prozeß erschwert. Die Anwesenheit von Wasser führt hingegen zu einem Isophoronverlust und zu aufwendigen Aufarbeitungsschritten.

Der vorliegenden Erfindung lag daher die Aufgabe zugrunde, den zuvor genannten Nachteilen abzuhelfen.

Demgemäß wurde ein neues und verbessertes Verfahren zur Herstellung von 3-Cyano-3,5,5-trimethylcyclohexanon durch Umsetzung von Isophoron mit Cyanwasserstoff in Gegenwart von quartären Ammoniumkatalysatoren bei Temperaturen von 80 bis 180°C und Drücken von 0,5 bis 20 bar gefunden, welches dadurch gekennzeichnet ist, daß man als Ammoniumkatalysatoren Salze der allgemeinen Formel I 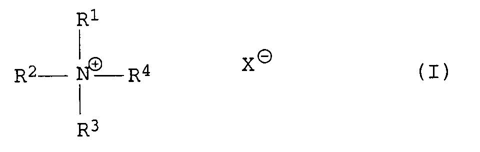 in der
- R¹, R², R³, R⁴: C₁- bis C₁₈-Alkyl, C₅- bis C₈-Cycloalkyl, Aryl, C₇- bis C₁₈-Aralkyl oder C₂- bis C₁₈-Hydroxyalkyl und
- X: 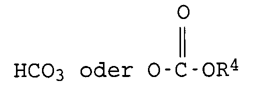
bedeuten, mit der Maßgabe, daß R⁴ für C₁- bis C₈-Alkyl steht, wenn X 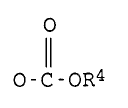 ist, einsetzt und mit der Maßgabe, daß im Fall von X = HCO₃ der Katalysator nicht in situ aus Natrium- oder Kaliumhydrogencarbonat und einem quartären Ammoniumhalogenid hergestellt wird.

Das erfindungsgemäße Verfahren läßt sich wie folgt durchführen:

Man kann beispielsweise
a) Isophoron mit dem quartären Ammoniumkatalysatoren vorlegen und Cyanwasserstoff in einem inerten Lösungsmittel oder in Isophoron zugeben oder
b) Isophoron mit Cyanwasserstoff vorlegen und den quartären Ammoniumkatalysator in einem inerten Lösungsmittel oder Isophoron zugeben oder
c) Isophoron vorlegen und Cyanwasserstoff und den quartären Ammoniumkatalysator in einem inerten Lösungsmittel oder Isophoron zugeben.

Die vorgelegte Reaktionsmischung kann auf die Reaktionstemperatur von 80 bis 180°C, bevorzugt 100 bis 170°C, besonders bevorzugt 120 bis 140°C aufgeheizt werden. Der Reaktionsdruck betragt in der Regel 0,5 bis 20 bar, bevorzugt 0,9 bis 10 bar, besonders bevorzugt Atmosphärendruck (Normaldruck). Als Reaktionsgefäße bzw. Reaktoren eignen sich beispielsweise Rührreaktoren oder Mischkreise. Die erfindungsgemäße Umsetzung kann diskontinuierlich oder bevorzugt kontinuierlich, beispielsweise dadurch erfolgen, daß ein Amin der allgemeinen Formel II R¹R²R³N mit Dialkylcarbonaten (R⁴O-(C=O)-OR⁴) in Gegenwart von Isophoron und Cyanwasserstoff in einem Rührreaktor oder Mischkreis unter Normaldruck oder unter erhöhtem Druck (2 bis 10 bar) umgesetzt wird, durchgeführt werden.

Als quartäre Ammoniumkatalysatoren I eignen sich quartäre Ammoniumhydrogencarbonate (X = HCO₃) und bevorzugt quartäre Ammoniumalkyl-carbonate (X = O-(C=O)-OR⁴) in der Regel in Mengen von 0,005 bis 5 Mol-%, bevorzugt 0,01 bis 2 Mol-%, besonders bevorzugt 0,05 bis 1 Mol-% bezogen auf Isophoron.

Als Beispiele für die Ammoniumalkylcarbonate (X = O-(C=O)-OR⁴) als Katalysatoren seien genannt:

Tetramethylammoniummethylcarbonat, Methyltributylammoniummethylcarbonat, Tetraethylammoniummethylcarbonat, Benzyltrimethyl-ammoniummethylcarbonat, Methyltridodecylammoniummethylcarbonat, Tetrabutylammoniummethylcarbonat, Tetrabutylammoniummethylcarbonat, Methyltriethylammoniummethylcarbonat, Phenyltrimethylammoniummethylcarbonat, Phenyldimethylmethylammoniummethylcarbonat, Tris-(2-hydroxyethyl)-methylammoniummethylcarbonat, 2-Hydroxyethyl-trimethylammoniumethylcarbonat.

Als Beispiele für die Ammoniumhydrogencarbonate (X = HCO₃) als Katalysatoren seien genannt:

Tetramethylammoniumhydrogencarbonat, Methyltributylammoniumhydrogencarbonat, Tetraethylammoniumhydrogencarbonat, Benzyltrimethylammoniumhydrogencarbonat, Methyltridodecylammoniumhydrogencarbonat, Tetrabutylammoniumhydrogencarbonat, Tetrabutylammoniumhydrogen-carbonat, Methyltriethylammoniumhydrogencarbonat, Phenyltrimethyl-ammoniumhydrogencarbonat, Phenyldimethylmethylammoniumhydrogen-carbonat, Tris-(2-hydroxyethyl)-methylammoniumhydrogencarbonat, 2-Hydroxyethyl-trimethylammoniumhydrogencarbonat.

Die quartären Ammoniumalkylcarbonate (X = O-(C=O)-OR⁴) können nach EP-A-291 074 durch Reaktion etwa stöchiometrischer Mengen an tertiärem Amin mit Dialkylcarbonaten (R⁴O-(C=O)-OR⁴) hergestellt werden. Die entstehende Lösung kann direkt ohne einen weiteren Verfahrensschritt eingesetzt werden.

Mit diesen Katalysatoren sind besonders kurze Verweilzeiten bei guten Ausbeuten und Selektivitäten erzielbar. Die als Nebenreaktion bekannte Oligomerisierung von Isophoron wird nahezu vollständig vermieden und der zu entsorgende Destillationsrückstand dadurch minimiert.

Als inerte Lösungsmittel eignen sich Wasser und C₁- bis C₂₀-Alkanole, bevorzugt C₁- bis C₈-Alkanole, besonders bevorzugt C₁- bis C₄-Alkanole wie Methanol, Ethanol, n-Propanol, iso-Propanol, n-Butanol, iso-Butanol und tert.-Butanol, aliphatische Kohlenwasserstoffe mit 5 bis 30 C-Atomen, bevorzugt mit 5 bis 20 C-Atomen, besonders bevorzugt mit 5 bis 10 C-Atomen wie n-Pentan, Pentanisomerengemische, n-Hexan, Hexanisomerengemische, n-Heptan, Heptanisomerengemische, n-Octan, Octanisomerengemische, cycloaliphatische Kohlenwasserstoffe mit 5 bis 20 C-Atomen, bevorzugt mit 5 bis 12 C-Atomen, besonders bevorzugt mit 5 bis 8 C-Atomen wie Cyclopentan, Cyclohexan, Cycloheptan und Cyclooctan, polare Lösungsmittel wie Dimethylformamid, Dimethylacetamid, N-Methylpyrrolidon oder cyclische Harnstoffe.

Besonders bevorzugt verwendet man Isophoron als Lösungsmittel.

Zur Neutralisation des Reaktionsaustrages können Säuren z.B. anorganische Säuren wie Phosphorsäure und Schwefelsäure, organische Säuren, beispielsweise Sulfonsäuren wie Methansulfonsäure, Toluolsulfonsäure, Carbonsäuren wie Ameisensäure, Essigsäure, Propionsäure, Malonsäure und Adipinsäure verwendet werden.

Die Substituenten R¹, R², R³, R⁴ und X in den Verbindungen der allgemeinen Formel I haben folgende Bedeutungen:
- R¹, R², R³ und R⁴: - gleich oder verschieden
- C₁- bis C₁₈-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Di-methylpropyl, n-Hexyl, iso-Hexyl, sec.-Hexyl, n-Heptyl, iso-Heptyl, n-Octyl, iso-Octyl, besonders bevorzugt C₁-bis C₄-Alkyl wie Methyl, Ethyl, n-Propyl, iso-Propyl, n-Butyl, iso-Butyl, sec.-Butyl und tert.-Butyl,
- C₅- bis C₈-Cycloalkyl, bevorzugt Cyclopentyl, Cyclohexyl und Cyclooctyl, besonders bevorzugt Cyclopentyl und Cyclohexyl,
- Aryl wie Phenyl, 1-Naphthyl und 2-Naphthyl, bevorzugt Phenyl,
- C₇- bis C₁₈-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₂- bis C₁₈-Hydroxyalkyl, bevorzugt C₂- bis C₁₀-Hydroxyalkyl, besonders bevorzugt C₂ bis C₅-Hydroxyalkyl wie 1-Hydroxyethyl, 2-Hydroxyethyl, 1-Hydroxy-n-propyl, 2-Hydroxy-n-propyl, 3-Hydroxy-n-propyl.
- X: 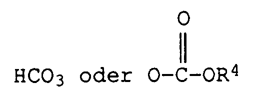
mit der Maßgabe, daß R⁴ für C₁- bis C₈-Alkyl, bevorzugt für C₁-bis C₄-Alkyl, besonders bevorzugt für Methyl und Ethyl steht, wenn X 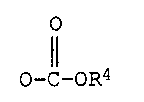 ist, einsetzt.

3-Cyano-3,5,5-trimethylcyclohexanon (Isophoronnitril) ist ein Zwischenprodukt zur Herstellung von Isophorondiamin, das als Epoxyhärter oder als Monomer für Polyamine und Polyurethane verwendet wird.

### Beispiele

### Beispiel 1

In einer Reaktionsapparatur mit Rührer, Kühler, Thermometer und Tropftrichter werden 622 g (4,5mol) Isophoron und 4,47 g (30 mmol) Tetramethylammoniummethylcarbonat vorgelegt. Innerhalb von 60 min werden bei 120°C 288,3 g einer Mischung aus Isophoron und Blausäure (3 mol HCN, 1,5 mol Isophoron) zugegeben. Nach Beendigung der Dosierung beträgt die HCN-Konzentration 20 ppm. Anschließend werden 3,5 g 85%iger H₃PO₄ zugegeben und der Reaktionsaustrag bei 0,1 mbar destilliert. Man erhält 426,6 g Isophoron (99 % bezogen auf umgesetztes Isophoron) und 476,8 g 3-Cyano-3,5,5-trimethylcyclohexanon (96,2 % bezogen auf eingesetzte HCN).

### Beispiel 2

622 g (4,5 Mol) Isophoron werden in Gegenwart von 4,14 g (0,02 mol) Butyltrimethylammoniummethylcarbonat mit 288,3 g einer Mischung aus 3 mol HCN und 1,5 mol Isophoron innerhalb von 120 min bei einer Temperatur von 115°C versetzt. Nach Neutralisation mit 4 g H₃PO₄ wird destilliert. Man erhält 424,3 g Isophoron und 478,3 g 3-Cyano-3,5,5-trimethylcyclohexanon. Dies entspricht einer Ausbeute von 98,8 % bezogen auf umgesetztes Isophoron und 96,5 % bezogen auf eingesetzte HCN.

### Beispiel 3

In einer 2-stufigen Rührkesselkaskade von je 250 ml Volumen werden bei Temperaturen von 120°C stündlich 102,9 g Isophoron, 10,06 g wasserfreier, stabilisatorhaltiger Cyanwasserstoff und 0,72 g einer 50 %igen methanolischen Tetramethylammoniummethylcarbonatlösung dosiert. Der Gehalt an HCN am Auslauf des 2. Reaktors beträgt 100 ppm. Das Reaktionsgemisch wird stündlich mit 0,25 g 85% iger H₃PO₄ versetzt und in einem Austragsgefäß gesammelt. Nach 24stündiger Vorlaufzeit werden 620 g des stabilisierten Rohgemisches einer fraktionierten Destillation zugeführt. Nach Abtrennung von 2,2 g Leichtsiedern werden bei einem Vakuum von 1 mbar 286,1 g Isophoron vom Siedepunkt 40 bis 42°C und 324,9 g 3-Cyano-3,5,5-trimethyl-cyclohexanon von Siedepunkt 94 bis 95°C abdestilliert. Das entspricht einer Ausbeute von 96,8 % bezogen eingesetzten Cyanwasserstoff und 98,8 % bezogen auf umgesetztes Isophoron.

Der Destillationsrückstand beträgt 6,9 g, das sind 1,2 % bezogen auf eingesetztes Rohprodukt.

### Beispiel 4

622 g (4,5 mol) Isophoron werden bei 120°C innerhalb von 120 min mit einer Mischung aus 207,3 g (1,5 mol) Isophoron und 81 g (3,0 mol) HCN in Gegenwart von 5,2 g (0,02 mol) einer 50 %igen wäßrigen Tetramethylammoniummethylcarbonatlösung versetzt. 10 min nach Dosierende beträgt die HCN-Konzentration nur noch 140 ppm. Nach Neutralisation mit 4 g H₃PO₄ wird destilliert. Man erhält nach einem Vorlauf von 3 g 422,6 g Isophoron und 476,7 g 3-Cyano-3,5,5-trimethylcyclohexanon. Dies entspricht einer Ausbeute von 96,2 % bezogen auf eingesetzte HCN und 98 % bezogen auf umgesetztes Isophoron.

### Beispiel 5

622 g (4,5 mol) Isophoron werden in Gegenwart von 4 g (0,021 mol) Triethylmethylammoniummethylcarbonat mit 288,3 g einer Mischung aus 3 mol HCN und 1,5 mol Isophoron innerhalb von 120 min bei einer Reaktionstemperatur von 120°C versetzt. Der HCN-Umsatz beträgt nach Reaktionsende 99,9 %. Nach der Neutralisation mit 2,4 g H₃PO₄ wird destilliert. Man erhält 424,1 g Isophoron und 479,2 g 3-Cyano-3,5,5-trimethylcyclohexanon; der Destillationsrückstand beträgt 12,8 g, dies entspricht 1,4 % bezogen auf eingesetztes Rohprodukt. Die 3-Cyano-3,5,5-trimethylcyclohexanonausbeute beträgt somit 96,7 %, die Isophoronselektivität 99 %.

### Vergleichsbeispiel

Zu 415 g (3 mol) Isophoron, das 12 g (0,077 mol) Tetraethylammoniumcyanid enthält, werden innerhalb von 1 Stunde 81 g (3 mol) Cyanwasserstoff bei einer Reaktionstemperatur von 108°C zugetropft. Nach einer Nachreaktionszeit von 30 min beträgt die HCN-Konzentration 0,16 %. Der Reaktionsaustrag wird mit 9 g H₃PO₄ neutralisiert und destilliert. Man erhält 15 g Isophoron und 443,6 g 3-Cyano-3,5,5-trimethylcyclohexanon. Das entspricht einer Ausbeute von 89,6 % bezogen auf eingesetzte HCN und 93 % bezogen auf umgesetztes Isophoron. Der Destillationsrückstand beträgt 28,2 g, das sind 5,5 % bezogen auf eingesetztes Rohprodukt.

## Patentansprüche

1. Verfahren zur Herstellung von 3-Cyano-3,5,5-trimethylcyclohexanon durch Umsetzung von Isophoron mit Cyanwasserstoff in Gegenwart von quartären Ammoniumkatalysatoren bei Temperaturen von 80 bis 180°C und Drücken von 0,5 bis 20 bar, dadurch gekennzeichnet, daß man als Ammoniumkatalysatoren Salze der allgemeinen Formel I 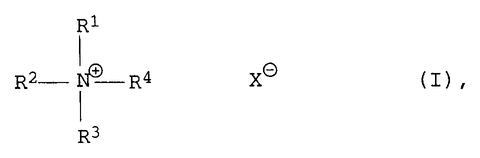 in der
R¹, R², R³, R⁴ C₁- bis C₁₈-Alkyl, C₅- bis C₈-Cycloalkyl, Aryl, C₇- bis C₁₈-Aralkyl oder C₂- bis C₁₈-Hydroxyalkyl und
X 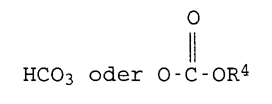
bedeuten, mit der Maßgabe, daß R⁴ für C₁- bis C₈-Alkyl steht, wenn X 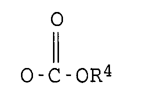 ist, einsetzt und mit der Maßgabe, daß im Fall von X = HCO₃ der Katalysator nicht in situ aus Natrium- oder Kaliumhydrogencarbonat und einem quartären Ammoniumhalogenid hergestellt wird.

2. Verfahren nach Anspruch 1, dadurch gekennzeichnet, daß man 0,005 bis 5 Mol-% eines Ammoniumkatalysators der Formel I einsetzt.

3. Verfahren nach Anspruch 1, dadurch gekennzeichnet, daß man die Umsetzung bei Temperaturen von 100 bis 170°C durchführt.

4. Verfahren nach Anspruch 1, dadurch gekennzeichnet, daß man die Umsetzung bei Drücken von 0,9 bis 10 bar durchführt.

5. Verfahren nach Anspruch 1, dadurch gekennzeichnet daß man die Umsetzung bei Atmosphärendruck durchführt.

## Claims

1. A process for preparing 3-cyano-3,5,5-trimethylcyclohexanone by reacting isophorone with hydrogen cyanide in the presence of quaternary ammonium catalysts at from 80 to 180°C and pressures of from 0.5 to 20 bar, wherein the ammonium catalysts used are salts of the formula I 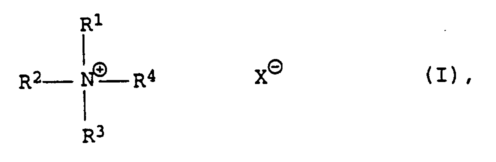 where
R¹, R², R³, R⁴ are C₁-C₁₈-alkyl, C₅-C₁₈-cycloalkyl, C₅-C₈-cycloalkyl, aryl, C₇-C₁₈-aralkyl or C₂-C₁₈-hydroxyalkyl and
X 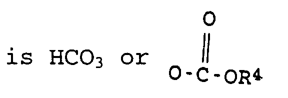
with the proviso that R⁴ is C₁-C₈-alkyl when X is 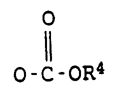 and with the proviso that when X = HCO₃, the catalyst is not prepared in situ from sodium or potassium hydrogen carbonate and a quaternary ammonium halide.

2. A process as claimed in claim 1, wherein from 0.005 to 5 mol% of an ammonium catalyst of the formula I is used.

3. A process as claimed in claim 1, wherein the reaction is carried out at from 100 to 170°C.

4. A process as claimed in claim 1, wherein the reaction is carried out at pressures of from 0.9 to 10 bar.

5. A process as claimed in claim 1, wherein the reaction is carried out at atmospheric pressure.

## Revendications

1. Procédé de préparation de 3-cyano-3,5,5-triméthylcyclohexanone par réaction d'isophorone avec de l'acide cyanhydrique en présence de catalyseurs ammonium quaternaire, à des températures de 80-180°C et sous des pressions de 0,5-20 bar, caractérisé en ce que l'on utilise, en tant que catalyseurs ammonium, des sels de formule générale I 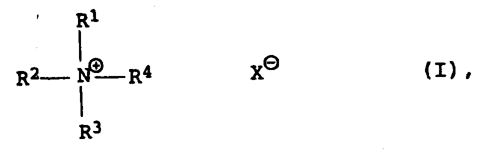 dans laquelle
R¹, R², R³, R⁴ représentent des groupements alkyle en C₁-C₁₈, cycloalkyle en C₅-C₈, aryle, aralkyle en C₇-C₁₈ ou hydroxyalkyle en C₂-C₁₈ et
X 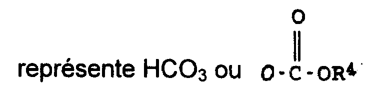
étant spécifié que R⁴ est mis pour un groupement allry'le en C₁-C₈ lorsque X représente 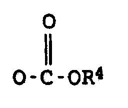 et étant spécifié que, lorsque X = HCO₃, le catalyseur n'est pas fabriqué in situ à partir d'hydrogénocarbonate de sodium ou de potassium et d'un halogénure d'ammonium quaternaire.

2. Procédé selon la revendication 1, caractérisé en ce que l'on utilise 0,005-5% en moles d'un catalyseur ammonium de formule I.

3. Procédé selon la revendication 1, caractérisé en ce que l'on mène la réaction à des températures de 100-170°C.

4. Procédé selon la revendication 1, caractérisé en ce que l'on mène la réaction sous des pressions de 0,9-10 bar.

5. Procédé selon la revendication 1, caractérisé en ce que l'on mène la réaction à pression atmosphérique.
